# EUROPÄISCHE PATENTSCHRIFT

(11) **EP 2 429 507 B1**
(45) Veröffentlichungstag und Bekanntmachung des Hinweises auf die Patenterteilung: **19.07.2017**
(21) Anmeldenummer: 10723922.0
(22) Anmeldetag: 14.05.2010
(51) Int. Cl.: A61K 31/00, A61K 31/194, A61K 31/5575, A61K 45/06, A61K 9/00, A61K 9/08, A61P 27/06, A61K 31/382, A61K 31/5377

(54) **PHOSPHATFREIE PHARMAZEUTISCHE ZUSAMMENSETZUNG ZUR GLAUKOMBEHANDLUNG**
PHOSPHATE-FREE PHARMACEUTICAL COMPOSITION FOR THE TREATMENT OF GLAUCOMA
COMPOSITION PHARMACEUTIQUE SANS PHOSPHATES POUR LE TRAITEMENT DU GLAUCOME

(30) Priorität: 14.05.2009 DE 102009021372
(43) Veröffentlichungstag der Anmeldung: 21.03.2012
(73) Patentinhaber: URSAPHARM Arzneimittel GmbH, 66129 Saarbrücken (DE)
(72) Erfinder: HOLZER, Frank, 66386 St. Ingbert (DE)
(74) Vertreter: Pfenning, Meinig & Partner mbB
(86) Internationale Anmeldenummer: PCT/EP2010/002981
(87) Internationale Veröffentlichungsnummer: WO 2010/130462

(56) Entgegenhaltungen:
- EP-A1- 1 312 366
- EP-A1- 1 985 298
- WO-A1-98/53809
- WO-A1-2008/096804
- WO-A2-01/58866
- DE-A1-102005 055 275
- US-A1- 2001 044 467
- US-A1- 2005 276 867
- US-A1- 2008 021 101
- NORBERT FRANZ SCHRAGE ET AL: "Relationship of eye burns with calcifications of the cornea?" GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE LNKD- DOI:10.1007/S00417-004-1089-2, Bd. 243, Nr. 8, 1. August 2005 (2005-08-01), Seiten 780-784, XP019342832 ISSN: 1435-702X
- R Roswell: "Sodium citrate reduces the incidence of corneal ulcerations and perforations in extreme alkali-burned eyes - acetylcysteine and ascorbate have no favorable effect", Invest. Ophthalmol. Vis. Sci, 1 September 1981 (1981-09-01), XP055310640, Retrieved from the Internet: URL:http://iovs.arvojournals.org/data/Jour nals/IOVS/933330/486.pdf [retrieved on 2016-10-13]
- AHMED I ET AL: "Evaluation of buffer systems in ophthalmic product development", INTERNATIONAL JOURNAL OF PHARMACEUTICS, ELSEVIER BV, NL, vol. 44, no. 1-3, 1 June 1988 (1988-06-01) , pages 97-105, XP025554378, ISSN: 0378-5173, DOI: 10.1016/0378-5173(88)90105-6 [retrieved on 1988-06-01]

## Beschreibung

Die Erfindung betrifft eine phosphatfreie pharmazeutische Zusammensetzung, die wenigstens einen FP-Prostanoidrezeptor-Agonisten und/oder wenigstens einen Prostamidrezeptor-Agonisten sowie Citratsalze und/oder Citronensäure umfasst, zur Anwendung in einem Verfahren gemäß den Ansprüchen. Glaukome, die auch als grüner Star bezeichnet werden, können aufgrund des erhöhten Augeninnendruckes zu einem Verlust von retinalen Ganglienzellen und Sehnervenfasern bis zur vollständigen Erblindung führen. Der sich in dem Augeninneren aufbauende Druck ist auf eine Beeinträchtigung des Kammerwasserabflusses aus der Vorderkammer und der Hinterkammer des Auges zurückzuführen. Normalerweise verlässt das vom Ziliarkörperepithel sekretierte Kammerwasser das Auge über den uveoscleralen und den trabekulären Abfluss.

Zur Therapie von Glaukom werden beispielsweise Parasympathomimetika, wie beispielsweise Pilocarpin oder Sympathomimetika, wie beispielsweise Dipivefrin, verwendet.

Auch werden zur Senkung des Augeninnendruckes Betablocker, wie Timolol, und Carboanhydrasehemmer, wie Dorzolamid, die eine Drosselung des Zuflusses bzw. eine Reduktion der Kammerwasserproduktion bewirken, verwendet.

Seit einigen Jahren werden auch Prostaglandinanaloga, wie Latanoprost, Tafluprost und Travoprost, sowie das Prostamid Bimatoprost bei der Therapie von Glaukomen verwendet.

Bei der Therapie eines Glaukoms handelt es sich in der Regel um eine Langzeitbehandlung.

Aus der DE102005055275 ist die Verwendung von mindestens einem Calcium-Chelatbildner und mindestens einem ophthalmologischen Viskositätsregler zur Herstellung einer phosphatfreien pharmazeutischen Zusammensetzung zur Behandlung und/oder Prävention von Epitheldefekten in der Hornhaut und/oder Bindehaut des Auges bekannt.

Es hat sich nunmehr herausgestellt, dass es bei Langzeitbehandlungen von Glaukompatienten durch topische Applikation von herkömmlichen Augentropfen oder Augensprays auf der Augenoberfläche zu einer Beeinträchtigung des Sehvermögens durch Trübung der Hornhaut kommen kann. Diese Trübung der Hornhaut kann durch Ein- und/oder Ablagerungen von schwer löslichen Calciumphosphaten, die sich in oder auf der Hornhaut sowie der Bindehaut des Auges einlagern oder ablagern, hervorgerufen werden. Diese Degeneration der Hornhaut des Auges bezeichnet man auch als Hornhautbanddegeneration oder bandförmige Keratopathie. Bereits geringfügige Ein- und/oder Ablagerungen von schwer löslichen Calciumphosphaten in bzw. auf der Hornhaut des Auges führen zu einer massiv erhöhten Blendempfindlichkeit, die auf eine an den Ab- oder Einlagerungen von Calciumphosphat(en) oder von schwer löslichen Calciumverbindungen erfolgende Lichtstreuung zurückzuführen ist. Insbesondere wird das Sehvermögen in der Nacht hierdurch stark beeinträchtigt.

Mithin besteht ein Bedarf an einer pharmazeutischen Zusammensetzung, die eine Langzeitbehandlung von Glaukom ohne die zuvor genannten Nebenwirkungen ermöglicht.

Die der Erfindung zugrunde liegende Aufgabe wird durch Bereitstellung einer phosphatfreien pharmazeutischen Zusammensetzung gelöst, wobei die phosphatfreie pharmazeutische Zusammensetzung wenigstens einen FP-Prostanoidrezeptor-Agonisten und/oder wenigstens einen Prostamidrezeptor-Agonisten sowie Citratsalze und/oder Citronensäure umfasst, zur Verhinderung oder Prophylaxe der Ab- oder Einlagerung von schwer löslichen Calcium-Verbindungen auf oder in der Hornhaut und/oder der Bindehaut des Auges.

Durch die phosphatfreie pharmazeutische Zusammensetzung wird die Bildung schwer löslicher Calcium-Verbindungen, Calcium-Phosphat-Komplexe und/oder Calcium-Phosphat-Verbindungen im Auge verhindert. Die Zusammensetzung ist weiter für die Prophylaxe der Calcifikation und/oder Verkalkung der Hornhaut und/oder der Bindehaut des Auges geeignet.

Die der Erfindung zugrunde liegende Aufgabe wird des Weiteren durch die Verwendung der erfindungsgemäßen phosphatfreien Zusammensetzung zur Herstellung eines Medikaments zur Therapie und/oder Prävention von Glaukom gelöst.

Bevorzugte Weiterbildungen sind in den Unterarsprüchen angegeben. Gegenstände, die nicht durch den Schutzumfang der Ansprüche umfasst sind, sind nicht Teil der beanspruchten Erfindung.

Unter einem FP-Prostanoidrezeptor werden Rezeptoren verstanden, an die Prostaglandin F₂ₐₗₚₕₐ-Analoga und/oder das Prostaglandin F₂ₐₗₚₕₐ bindet.

Unter dem Begriff FP-Prostanoidrezeptor werden erfindungsgemäß auch FP-Rezeptoren verstanden, an die Prostaglandin F₂ₐₗₚₕₐ binden. Bei den FP-Rezeptoren handelt es sich um G-Protein-gekoppelte Rezeptoren, deren endogener physiologischer Aktivator das Prostaglancin F₂ₐₗₚₕₐ ist. Bei einer Stimulierung des FP-Prostanoidrezeptors kommt es zu einer Verstärkung des Kammerwasserabflusses insbesondere über den uveoscleralen Weg.

Neben der Stimulierung des FP-Prostanoidrezepters kann ein Kammerwasserabfluss aus dem Augeninneren auch durch eine Stimulierung des Prostamidrezeptors bewirkt werden. Bei den Prostamiden handelt es sich um Prostaglandin F₂ₐₗₚₕₐ-1-amide. Ohne an eine Theorie gebunden sein zu wollen, wird davon ausgegangen, dass Prostamid an den Prostamidrezeptor und/oder den Prostanoidrezeptor bindet und zu einem Abfluss des Kammerwassers aus dem Augeninneren, insbesondere über den uveoscleralen Weg, führt.

Die Erfinder haben nun überraschend festgestellt, dass die topische Applikation wenigstens eines FP-Prostanoidrezeptor-Agonisten und/oder wenigstens eines Prostamidrezeptor-Agonisten in Kombination mit Citratsalzen und/oder Citronensäure eine völlig überraschende Langzeitbehandlung des Auges ermöglicht, ohne dass dabei die unerwünschten Nebenwirkungen einer Calcifikation oder Verkalkung der Hornhaut und/oder der Bindehaut des Auges eintritt.

Citrate, d.h. Salze der Citronensäure, und Citronensäure wirken unter anderem als Calcium-Chelatbildner. Da die erfindungsgemäße pharmazeutische Zusammensetzung phosphatfrei ist, wird zum einen kein zusätzliches Phosphat auf die Augenoberfläche aufgebracht. Zum anderen werden Calciumionen, insbesondere Ca²⁺-Ionen, von der Citronensäure bzw. den Citraten komplexiert, so dass der Bildung schwer löslicher Calciumphosphate und/oder Calciumverbindungen entgegengewirkt wird bzw. deren Bildung vorzugsweise verhindert wird.

Herkömmlicherweise sind Augentropfen oder Augenlösungen vorzugsweise phosphatgepuffert, da der Phosphatpuffer ein sehr stabiles Puffersystem mit sehr guter Pufferkapazität ist, das insbesondere sehr lagerstabil ist.

Citronensäure bzw. Citratsalze bilden nach Lösung in einem wässrigen Medium, vorzugsweise in Wasser, ebenfalls ein stabiles Puffersystem mit ausreichender Pufferkapazität, obwohl die Pufferkapazität im Vergleich zu einem Phosphatpuffer schwächer ist. Vorzugsweise liegt der pH-Wert der erfindungsgemäßen pharmazeutischen Zusammensetzung in einem physiologischen Bereich, vorzugsweise in einem Bereich von pH 5,5 bis 8,5, vorzugsweise 5,8 bis 7,8, weiter bevorzugt von 6,0 bis 7,2. Der pH-Wert wird durch Zugabe von Säure bzw. Lauge, vorzugsweise 0,1 N HCl oder 0,1 N NaOH, gegebenenfalls eingestellt.

Zur Herstellung des Citratpuffers können sowohl Citronensäure, primäre, sekundäre und/oder tertiäre Citrate verwendet werden. Als Citrate werden vorzugsweise Alkalimetallcitrate, weiter bevorzugt Natriumcitrate, verwendet. Vorzugsweise werden Citronensäure, Natriumcitrat, Dinatriumcitrat und/oder Trinatriumcitrat verwendet. Der Citratpuffer liegt vorzugsweise in einer Konzentration von 5 mmol/l bis 100 mmol/l, weiter bevorzugt von 10 mmol/l bis 50 mmol/l vor.

Gemäß einer äußerst bevorzugten Ausführungsform ist die phosphatfreie pharmazeutische Zusammensetzung auch Calciumionen-frei. Calciumionen-frei im Sinne der Erfindung bedeutet, dass die phosphatfreie pharmazeutische Zusammensetzung weniger als 0,3 mmol/l Calcium-Ionen, vorzugsweise weniger als 0,1 mmol/l Calcium-Ionen, und insbesondere bevorzugt keine Calcium-Ionen enthält.

Unter phosphatfrei wird im Sinne der Erfindung verstanden, dass die pharmazeutische Zusammensetzung weniger als 7 mmol/l Phosphat-Ionen, vorzugsweise weniger als 3 mmol/l Phosphat-Ionen, besonders bevorzugt weniger als 1 mmol/l Phosphat-Ionen und äußerst bevorzugt keine Phosphat-Ionen enthält.

Unter dem Begriff "Phosphat-Ionen" im Sinne der Erfindung werden insbesondere PO₄³⁻, HPO₄²⁻ und/oder H₂PO₄⁻ verstanden.

Die erfindungsgemäße phosphatfreie pharmazeutische Zusammensetzung verhindert aufgrund der Abwesenheit von Phosphat-Ionen und aufgrund des Vorhandenseins von Citratsalzen und/oder Citronensäure die Bildung von Calcium-Phosphat-Komplexen und/oder Calcium-Phosphat-Verbindungen und/oder anderen schwer löslichen Calcium-Verbindungen im Auge, die zur Ab- oder Einlagerung auf oder in der Hornhaut und/oder der Bindehaut des Auges und mithin zu einer erheblichen Einschränkung des Sehvermögens durch Lichtstreuung an den Calcium-Phosphat-Komplexen und/oder Calcium-Phosphat-Verbindungen und/oder anderen schwer löslichen Calcium-Verbindungen führen können. Unter schwer löslichen Calcium-Verbindungen werden insbesondere Verbindungen verstanden, die in der Hornhaut Ab- oder Einlagerungen bilden.

Die Citratsalze und/oder Citronensäure komplexieren auch im Auge physiologisch vorhandene Calcium-Ionen und wirken somit der Entstehung schwer löslicher Calciumverbindungen entgegen, die zu Ab- oder Einlagerungen in der Hornhaut und/oder Bindehaut des Auges führen können.

Die erfindungsgemäße phosphatfreie pharmazeutische Zusammensetzung verhindert oder verringert eine Calcifikation in der Hornhaut und/oder in der Bindehaut des Auges.

Ferner wirken die Citratsalze und/oder Citronensäure bzw. der daraus hergestellte Citratpuffer überraschenderweise auch wundheilungsfördernd. Somit unterstützt die erfindungsgemäße phosphatfreie pharmazeutische Zusammensetzung die Regeneration von etwaigen z.B. durch Konservierungsmittel und/oder einen insuffizienten Tränenfilm hervorgerufenen Defekten der Augenoberfläche, insbesondere der Hornhautoberfläche und/oder der Bindehaut des Auges.

Es hat sich gezeigt, dass die Citronensäure bzw. die Citrate aufgrund ihrer Komplexierungseigenschaften, insbesondere durch die Komplexierung von Calciumionen, eine verbesserte Penetration der FP-Prostanoide bzw. Prostamide von der epithelialen Seite in und durch die Cornea bewirken. Es wird vermutet, dass es aufgrund der Komplexierung von Calciumionen zu Veränderungen des intrazellulären Matrixmateriales der Cornea kommt. Diese Veränderungen bewirken vermutlich eine Lockerung der Tight Junctions, die auch als Zonula occludens bezeichnet werden. Die Lockerung der Tight Junctions ermöglicht sodann, dass die relativ hydrophoben FP-Prostanoide bzw. Prostamide leichter in und durch die Cornea in das Augeninnere gelangen können.

Die Verwendung von Citronensäure bzw. Citraten in Kombination mit FP-Prostanoid(en) und/oder Prostamid(en) führt mithin zu einer überraschenden synergistischen Wirkung. Zum einen wirken die Citronensäure und/oder Citrate bei einer, beispielsweise aufgrund des bei älteren Patienten häufiger auftretenden Krankheitsbildes des Trockenen Auges und/oder durch Verwendung konservierungsmittelhaltiger Augentropfen, vorgeschädigten Cornea durch die wundheilungsfördernden Eigenschaften entgegen. Zum anderen bewirken die Citronensäure und/oder die Citrate aufgrund der Lockerung der Tight Junctions durch die Komplexierung von Calciumionen eine verbesserte Aufnahme der FP-Prostanoide bzw. Prostamide in das Auge. Hierdurch wird zum einen die für die Aufnahme der FP-Prostanoide und/oder Prostamide erforderliche Verweilzeit der FP-Prostanoide bzw. Prostamide auf der Cornea verringert. Hierdurch wird insbesondere auch die Möglichkeit einer durch den natürlichen Tränenfluss des Auges bedingten Auswaschung der FP-Prostanoide und/oder Prostamide von der Augenoberfläche verringert und damit die Bioverfügbarkeit der FP-Prostanoide bzw. Prostamide im Augeninnere erhöht. Die verbesserte Aufnahme der FP-Prostanoide bzw. Prostamide ermöglicht es, die Konzentration der auf die Cornea aufzubringenden Menge an FP-Prostanoiden bzw. Prostamiden zu verringern.

Bei den vorgenannten FP-Prostanoiden handelt es sich insbesondere um Latanoprost, Travoprost und/oder Tafluprost, und bei den vorgenannten Prostamiden handelt es sich insbesondere um Bimatoprost.

Diese synergistische Wirkung von Citronensäure bzw. Citraten in Verbindung mit FP-Prostanoiden bzw. Prostamiden ist unerwartet. Hierdurch wird eine Reizung der Augenoberfläche verringert bzw. vermieden.

Die Erfindung betrifft auch die Verwendung von FP-Prostanoiden, insbesondere von Latanoprost, Travoprost und/oder Tafluprost, und/oder von Prostamid, insbesondere von Bimatoprost, in Kombination mit Citronensäure und/oder Citrat zur Bereitstellung einer erhöhten Konzentration des/der genannten Wirkstoffs/Wirkstoffe im Kammerwasser des Auges, in der Iris und/oder im Ziliarkörper.

Mithin hat sich überraschend herausgestellt, dass die erfindungsgemäße phosphatfreie pharmazeutische Zusammensetzung für das Auge sehr gut verträglich ist, insbesondere keine wesentliche Reizung des Auges bzw. der Augenoberfläche hervorruft.

Der FP-Prostanoidrezeptor-Agonisten und/oder der Prostamidrezeptor-Agonisten werden vorzugsweise in einer Konzentration in einem Bereich von 0,00001 Gew.-% bis 0,05 Gew. -% , weiter bevorzugt von 0,00005 Gew.-% bis 0,01 Gew.-%, noch weiter bevorzugt von 0,0001 Gew.-% bis 0,005 Gew.-%, jeweils bezogen auf das Gesamtgewicht der Zusammensetzung, verwendet.

Die erfindungsgemäße phosphatfreie pharmazeutische Zusammensetzung ermöglicht eine Absenkung des Augeninnendrucks in einem Bereich von 20 bis 40 %, üblicherweise in einem Bereich von 20 bis 30 %, bezogen auf den Augeninnendruck vor der Applikation.

Die Häufigkeit der Applikation der erfindungsgemäßen phosphatfreien pharmazeutischen Zusammensetzung erfolgt in Abhängigkeit von dem individuellen Bedarf bzw. der Schwere des Glaukoms. So können im Falle von Augentropfen 1 bis 3 Tropfen pro Auge, vorzugsweise einmal täglich, ausreichend sein. Bei schwerer Glaukomerkrankung können auch mehr Tropfen, beispielsweise bis zu 16 Tropfen, auch mehrmals täglich, pro Auge aufgebracht werden.

Gemäß einer weiteren bevorzugten Ausführungsform handelt es sich bei dem wenigstens einen FP-Prostanoidrezeptor-Agonisten und/oder dem wenigstens einen Prostamidrezeptoragonisten um Verbindungen mit der Strukturformel (I) oder (II) : 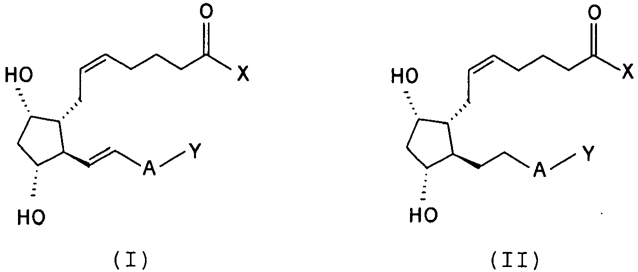 wobei
X unabhängig voneinander für OR₃ oder NR₁R₂ steht, wobei R₁, R₂, R₃ unabhängig voneinander für H, unverzweigten oder verzweigten Alkylrest mit 1 bis 8 Kohlenstoffatomen, vorzugsweise 2 bis 6 Kohlenstoffatomen, unverzweigten oder verzweigten OH-substituierten Alkylrest mit 1 bis 8 Kohlenstoffatomen, vorzugsweise 2 bis 6 Kohlenstoffatomen, steht;
A unabhängig voneinander für CHOH, C=O oder CF₂ steht;
Y unabhängig voneinander
   für unverzweigten oder verzweigten Alkylrest mit 1 bis 10 Kohlenstoffatomen, vorzugsweise 3 bis 8 Kohlenstoffatomen steht,
   oder für unverzweigten oder verzweigten Alkylarylrest mit 6 bis 12 Kohlenstoffatomen, vorzugsweise mit 7 bis 10 Kohlenstoffatomen, wobei Aryl vorzugsweise Phenyl ist, steht,
   oder für Trifluormethyl-substituierten Alkylarylrest mit 7 bis 12 Kohlenstoffatomen, vorzugsweise mit 8 bis 9 Kohlenstoffatomen, wobei Aryl vorzugsweise Phenyl ist, steht,
   oder für 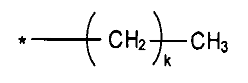 wobei k für eine ganze Zahl von 0 bis 9, vorzugsweise 2 bis 7, steht,
   oder für 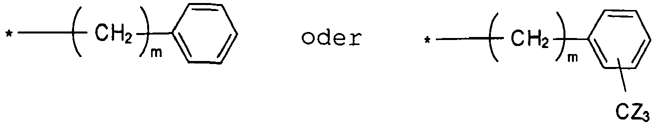 wobei m für eine ganze Zahl von 0 bis 6, vorzugsweise 2 bis 4, und wobei Z für H oder F steht,
   oder für 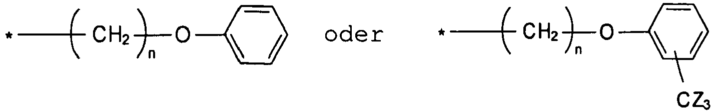 wobei n für eine ganze Zahl von 0 bis 4, vorzugsweise 1 bis 3, und wobei Z für H oder F steht.

Die vorstehend angegebenen FP-Prostanoidrezeptor-Agonisten bzw. Prostamidrezeptor-Agonisten können auch als pharmazeutische annehmbare Salze und/oder Ester vorliegen.

Gemäß einer bevorzugten Ausführungsform der Erfindung wird der FP-Prostanoidrezeptor-Agonist aus der Gruppe, die aus Prostaglandin, Prostaglandinanalogon und Gemischen davon besteht, ausgewählt.

Gemäß einer bevorzugten Weiterbildung der Erfindung handelt es sich bei dem Prostaglandin um Prostaglandin F2ajpha mit der Strukturformel (III). Das Prostaglandin F₂ₐₗₚₕₐ wird auch als Dinoprost bezeichnet:

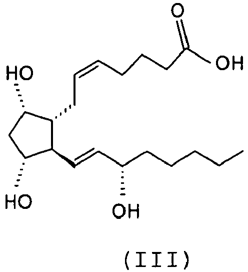

Das Prostaglandin F₂ₐₗₚₕₐ bzw. Dinoprost kann auch als pharmazeutisch annehmbarer Ester, beispielsweise als Alkylester mit einem Alkylrest von 1 bis 6 Kohlenstoffatomen vorliegen. Vorzugsweise handelt es sich bei dem Alkylester um einen Ethylester oder Isopropylester. Als sehr geeignet hat sich der Isopropylester erwiesen.

Gemäß einer weiteren bevorzugten Ausführungsform handelt es sich bei dem Prostaglandinanalogon um ein Prostaglandin F₂ₐₗₚₕₐ-Analogon.

Gemäß einer weiteren bevorzugten Ausführungsform wird das Prostaglandin F₂ₐₗₚₕₐ-Analogon aus der Gruppe, die aus Latanoprost (Strukturformel (IV)), Tafluprost (Strukturformel (V)), Travoprost (Strukturformel (VI)), Unoproston (Strukturformel (VII)), und Mischungen sowie deren pharmazeutisch annehmbaren Salzen und Estern davon besteht, ausgewählt:

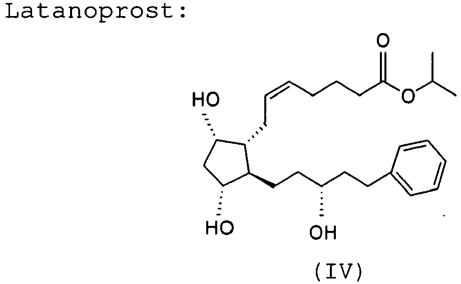

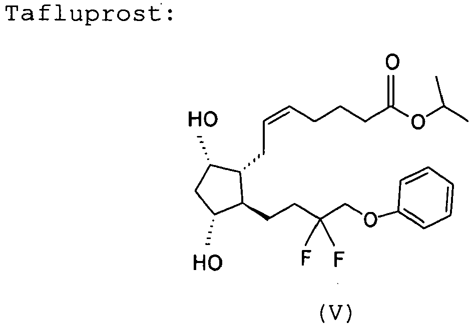

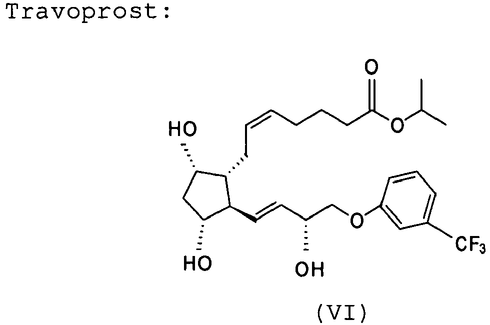

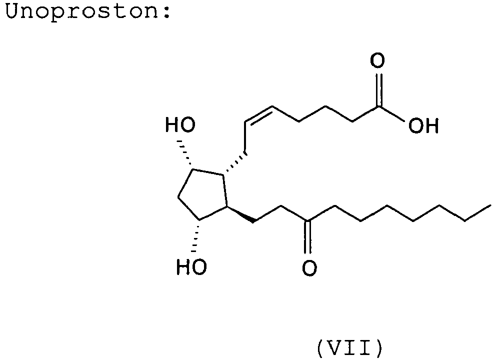

Das Unoproston kann auch als Alkylester vorliegen. Gemäß einer bevorzugten Ausführungsform handelt es sich hierbei um einen Ethyl- oder Isopropylester.

Gemäß einer weiteren Variante der Erfindung ist das Prostaglandin F₂ₐₗₚₕₐ-Analogon ein 15-Keto-Prostaglandin F₂ₐₗₚₕₐ-Analogon und wird vorzugsweise aus der Gruppe, die aus 15-Keto-Latanoprost, 15-Keto-Travoprost und Mischungen sowie deren pharmazeutisch annehmbaren Salzen und Estern davon besteht, ausgewählt. Bei dieser Variante ist die OH-Gruppe am C₁₅ im Prostaglandin F₂ₐₗₚₕₐ-Analogon durch eine Ketogruppe ersetzt.

Das Prostaglandin F₂ₐₗₚₕₐ kann gemäß einer Variante der Erfindung als Amid oder aber auch als pharmazeutisch annehmbares Salz davon vorliegen. Das pharmazeutisch annehmbare Salz kann beispielsweise ein Chlorid, Acetat, Sulfat, oder gemischte Salze davon, etc. sein. Das Prostaglandin F₂ₐₗₚₕₐ-amid auch als Prostamid bezeichnet.

Gemäß einer weiteren bevorzugten Ausführungsform ist der Prostamidrezeptor-Agonist Prostamid oder ein Prostamidanalogon.

Vorzugsweise ist das Prostamid Prostaglandin F₂ₐₗₚₕₐ-amid und weist folgende Strukturformel (VIII) auf: 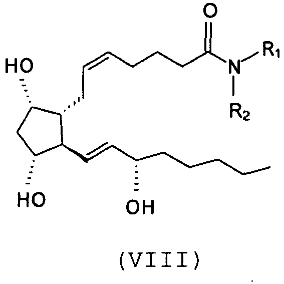 wobei R₁ und R₂ unabhängig voneinander für Wasserstoff, Alkyl oder Hydroxyalkyl mit 1 bis 8 Kohlenstoffatomen, vorzugsweise 2 bis 4 Kohlenstoffatomen stehen.

Die Substituenten R₁ und R₂ sind vorzugsweise unabhängig voneinander Wasserstoff, Methyl, Ethyl, n-Propyl, Isopropyl, n-Butyl, sec.-Butyl, n-Pentyl, n-Hexyl, n-Heptyl oder n-Octyl. Gemäß einer weiteren bevorzugten Ausführungsform sind die vorgenannten bevorzugten Alkylgruppen mit wenigstens einer OH-Gruppe substituiert. Vorzugsweise ist die OH-Gruppe endständig, d.h. an dem dem Stickstoff entfernt liegenden Ende der Alkylgruppe angeordnet.

Gemäß einer weiteren bevorzugten Ausführungsform ist R₂ Wasserstoff und R₁ ist Alkyl oder Hydroxyalkyl mit 1 bis 8 Kohlenstoffatomen, vorzugsweise mit 2 bis 4 Kohlenstoffatomen. R₁ ist dabei vorzugsweise Methyl, Ethyl, n-Propyl, Isopropyl, n-Butyl, sec.-Butyl, n-Pentyl, n-Hexyl, n-Heptyl oder n-Octyl. Gemäß einer weiteren bevorzugten Ausführungsform sind die vorgenannten bevorzugten Alkylgruppen mit wenigstens einer OH-Gruppe substituiert. Vorzugsweise ist die OH-Gruppe endständig, d.h. an dem dem Stickstoff entfernt liegenden Ende der Alkylgruppe R₁ angeordnet.

Das substituierte Amid kann dabei jeweils auch als pharmazeutisch annehmbares Salz oder annehmbarer Ester davon vorliegen. Das pharmazeutisch annehmbare Salz kann beispielsweise ein Chlorid, Acetat, Sulfat, oder gemischte Salze davon, etc. sein.

Gemäß einer weiteren bevorzugten Ausführungsform handelt es sich bei dem Prostaglandin F₂ₐₗₚₕₐ-Amid um Bimatoprost (Strukturformel (IX)) oder um Prostaglandin F₂ₐₗₚₕₐ-1-Ethanolamid (Strukturformel (X)) oder oder deren pharmazeutisch annehmbare Salze oder Ester. 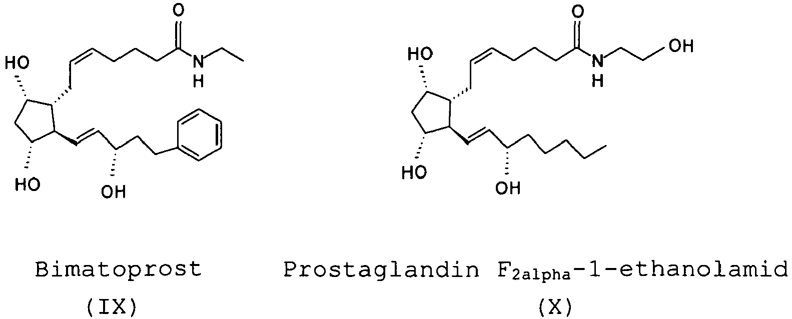 Gemäß einer weiteren Variante der Erfindung ist das Prostaglandin F₂ₐₗₚₕₐ-amid-Analogon ein 15-Keto-Prostaglandin F₂ₐₗₚₕₐ-amid-Analogon und wird vorzugsweise aus der Gruppe, die aus 15-Keto-Bimatoprost, 15-Keto-Prostaglandin F₂ₐₗₚₕₐ-1-ethanolamid und Mischungen sowie deren pharmazeutisch annehmbaren Salzen und Estern davon besteht, ausgewählt. Bei dieser Variante ist die OH-Gruppe am C₁₅ im Prostaglandin F₂ₐₗₚₕₐ-amid-Analogon durch eine Ketogruppe ersetzt.

Gemäß einer bevorzugten Weiterbildung umfasst die erfindungsgemäße phosphatfreie pharmazeutische Zusammensetzung mindestens einen ophthalmologisch verträglichen Viskositätsregler.

Als Viskositätsregler im Sinne der Erfindung werden Stoffe bezeichnet, die eine Viskositäts-erhöhende Wirkung haben.

Im Sinne der Erfindung wird unter "ophthalmologisch verträglich" insbesondere verstanden, daß keine Reizungen des Auges und vorzugsweise keine Beeinträchtigungen des Sehvermögens auftreten.

Vorzugsweise weist der Viskositätsregler ein viskoelastisches Verhalten auf. Unter einem viskoelastischen Verhalten wird erfindungsgemäß verstanden, daß sich die Viskosität unter der Einwirkung von Druck-, Zug-, Schub-, und/oder Scherspannungen ändert. Besonderes bevorzugt weist die erfindungsgemäße phosphatfreie pharmazeutische Zusammensetzung aufgrund des Viskositätsreglers das Verhalten einer Nicht-Newtonschen-Flüssigkeit auf.

Die Viskosität liegt vorzugsweise in einem Bereich von 2 bis 1000 mPa • 5, weiter bevorzugt in einem Bereich von 2 bis 500 mPa • s, besonders bevorzugt in eine Bereich von 2 bis 100 mPa • s.

Die Viskositäts-erhöhende Wirkung bewirkt äußerst vorteilhaft, daß die auf die Augenoberfläche aufgebrachte phosphatfreie pharmazeutische Zusammensetzung eine erhöhte Verweildauer aufweist und verlangsamt von der Augenoberfläche wieder abfließt. Das Nicht-Newtonsche Fließverhalten des Viskositätsreglers bedingt eine für die Anwendung am Auge hervorragende Eigenschaft, dass nämlich die Viskosität mit zunehmender Schergeschwindigkeit abnimmt. Nach Aufbringung der phosphatfreien pharmazeutischen Zusammensetzung mit dem Viskositätsregler auf die Oberfläche des Auges wird über den Lidschlag des Augenliedes eine Scherspannung an die phosphatfreie pharmazeutische Zusammensetzung angelegt, wodurch die zunächst erhöhte Viskosität erniedrigt wird. Durch den Lidschlag des Augenlides erniedrigt sich die Viskosität, so daß sich ein gleichmäßiger Film auf der Oberfläche des Auges ausbildet. Nach dem Lidschlag erhöht sich die Viskosität, so daß der Film an der Augenoberfläche gut anhaftet und nur verlangsamt abläuft, wodurch die Verweildauer der erfindungsgemäßen phosphatfreien Zusammensetzung auf der Augenoberfläche erhöht wird.

Aufgrund der erhöhten Verweildauer auf der Augenoberfläche kann die Bioverfügbarkeit des wenigstens einen FP-Prostanoidrezeptor-Agonisten und/oder des wenigstens einen Prostamidrezeptor-Agonisten erhöht werden, da ein rasches Abfließen des wenigstens einen FP-Prostanoidrezeptor-Agonisten und/oder des wenigstens einen Prostamidrezeptor-Agonisten verhindert und mithin der zur Aufnahme der Wirkstoffe verfügbare Zeitraum verlängert wird.

Vorzugsweise wirkt der Viskositätsregler zugleich als Gleit- und Schmiermittel auf dem Auge. Die Gleit- und Schmierwirkung ist insbesondere dann vorteilhaft, wenn die Augenoberfläche, insbesondere die Hornhaut, bereits Verletzungen, insbesondere Epithelläsionen, aufweist. Somit ist die Verwendung eines Viskositätsreglers insbesondere dann vorteilhaft, wenn aufgrund einer Langzeitherapie mit herkömmlichen pharmazeutischen Zusammensetzungen bereits eine Epithelläsion erfolgt ist.

Gemäß einer bevorzugten Ausführungsform beträgt die Menge an Viskositätsregler etwa 0,005 Gew.-% bis etwa 5 Gew.-%, bevorzugt etwa 0,01 Gew.-% bis etwa 1 Gew.-%, jeweils bezogen auf das Gesamtgewicht der phosphatfreien pharmazeutischen Zusammensetzung.

Gemäß einer bevorzugten Weiterbildung der Erfindung wird der ophthalmologisch verträgliche Viskositätsregler aus der Gruppe, die aus Chondroitinsulfat, Polyacrylamid, Polyacrylsäure, Polyacrylharze, Polyethylenglykol, Cellulosederivate, Polysaccharide, Polyvinylpyrrolidon, Hyaluronsäure, Hyaluronate, Derivaten davon und Mischungen davon besteht, ausgewählt wird.

Als sehr geeignet haben sich Hyaluronsäure und deren Salze, die Hyaluronate, erwiesen.

Hyaluronsäure ist Bestandteil des Glaskörpers des Auges und stellt insofern keine für den menschlichen Organismus fremde Verbindung dar. Aus diesem Grund ist Hyaluronsäure aus immunologischer Sicht sehr gut verträglich. Darüber hinaus weist Hyaluronsäure bzw. Hyaluronat eine strukturelle Ähnlichkeit mit Mucin auf. Mucin bildet die unterste Schicht des dreischichtigen Tränenfilms und sorgt für eine optimale Benetzung der Hornhaut- und Bindehautepithelien.

Weiterhin weist Hyaluronsäure eine für die Anwendung am Auge hervorragende Eigenschaft auf, dass nämlich die Viskosität mit Zunehmen der Schwergeschwindigkeit abnimmt. Die Hyaluronsäure weist somit ein Nicht-Newtonsches Fließverhalten auf.

Hyaluronsäure bzw. deren Salze, die Hyaluronate bzw. insbesondere Natrium-Hyaluronat weist bzw. weisen hervorragende optische Eigenschaften auf, so daß es zu keiner Beeinträchtigung des Sehvermögens bei den behandelten Patienten kommt.

Die Hyaluronsäure bzw. Hyaluronat kann aus dem Glaskörper des Rinderauges oder aber auch aus Hahnenkämmen isoliert werden. Weiterhin kann Hyaluronsäure bzw. Hyaluronat auch in Bakterienstämmen in pharmazeutischer Qualität hergestellt werden. Als Salze der Hyaluronsäure können bspw. Kalium-, Natrium- und/oder Magnesiumhyaluronat verwendet werden. Besonders bevorzugt ist das Natrium-Hyaluronat.

Aufgrund dieser physikalischen Eigenschaften eignen sich wässrige Natrium-Hyaluronat-Lösungen und/oder Hyaluronsäure hervorragend als Gleit- und Schmiermittel mit guter Haftwirkung und verlängerter Verweilzeit auf den konjunktivalen und kornealen Epithelien ohne Beeinträchtigung der Sehleistung.

Gemäß einer weiteren Ausführungsform weist die Hyaluronsäure und/oder das Hyaluronat ein Molekulargewicht auf, das in einem Bereich von 50.000 bis 10.000.000 Dalton, bevorzugt von etwa 250.000 bis 5.000.000 liegt. Besonders bevorzugt beträgt das Molekulargewicht der Hyaluronsäure bzw. des Hyaluronats 50.000 bis 4.000.000 Dalton. Äußerst bevorzugt weist die Hyaluronsäure bzw. das Hyaluronat ein Molekulargewicht von etwa 1.500.000 bis 3.500.000 Dalton auf. Die Hyaluronsäure und/oder das Hyaluronat werden vorzugsweise in einer Konzentration von 0,01 bis 1,0 Gew.-%, weiter bevorzugt von 0,05 bis 0,8 Gew.-%, besonders bevorzugt von 0,08 bis 0,4 Gew.-%, jeweils bezogen auf das Gesamtgewicht der phosphatfreien pharmazeutischen Zusammensetzung, verwendet.

Das hohe Molekulargewicht der Hyaluronsäure bzw. des verwendeten Hyaluronates wie bspw. Natrium-Hyaluronat bewirkt eine hohe Viskoelastizität bei niedriger Konzentration. In der Lösung liegen die Molekülketten in zufälliger Anordnung knäuelartig vor. Unter dem Einfluß der durch die Bewegung des Augenlides ausgeübten Scherkräfte richten sich die Makromoleküle in etwa parallel aus. Diese Änderung in der dreidimensionalen Struktur unter dem Einfluß von Scherkräften dürfte maßgeblich für die hervorragenden viskoelastischen Eigenschaften sein.

Gemäß einer weiteren bevorzugten Ausführungsform enthält die pharmazeutische Zusammensetzung phosphatfreie pharmazeutische Hilfsstoffe die aus der Gruppe, die aus anorganischen Puffersubstanzen, organischen Puffersubstanzen, anorganischen Salzen, organischen Salzen, Lösungsmitteln, Lösungsvermittlern, Lösungsbeschleunigern, Salzbildnern, Viskositäts- und Konsistenzbeeinflussern, Gelbildnern, Emulgatoren, Solubilisatoren, Benetzern, Spreizmitteln, Anti-Oxidantien, Konservierungsmitteln, Füll- und Trägerstoffen, Osmolaritätsreglern sowie Mischungen davon besteht, ausgewählt werden.

Die anorganischen Puffersubstanzen werden vorzugsweise aus der Gruppe, die aus Borsäure, Natriumhydroxid, Natriumborat, Natriumcarbonat, Salzsäure, Natriumhydrogencarbonat und Mischungen davon besteht, ausgewählt.

Die organischen Puffersubstanzen werden vorzugsweise aus der Gruppe, die aus Essigsäure, Natriumacetat, Kaliumhydrogenphthalat, Bernsteinsäure, Maleinsäure, Trometamol und Mischungen davon besteht, ausgewählt.

Die anorganischen Salze werden vorzugsweise aus der Gruppe, die aus Kochsalz, Kaliumchlorid, Aluminiumhydroxid, Ammoniumhydroxid, Ammoniumchlorid, Ammoniumsulfat, Calciumchlorid und Mischungen davon besteht, ausgewählt.

Die organischen Salze werden vorzugsweise aus der Gruppe, die aus Salzen der Bernsteinsäure, Salzen der Maleinsäure, Salzen der Essigsäure und Mischungen davon besteht, ausgewählt.

Die Emulgatoren, Solubilisatoren, Benetzungsmittel und Spreitmitttel werden vorzugsweise aus der Gruppe, die aus Poloxamer, Phospholipiden, Lecithin, Alkaliseifen, beispielsweise Natriumpalmitat, Alkalisulfate, beispielsweise Natriumlaurylsulfat, Macrogole, beispielsweise Polyethylenglykole, Macrogolstearate, Polysorbate, Macrogolglycerolmonostearate, Propylenglycole, Glycerin, Cyclodextrine und Mischungen davon besteht, ausgewählt.

Die Antioxidantien werden vorzugsweise aus der Gruppe, die aus Ascorbinsäure, Butylhydroxytoluol, Alpha-Tocopherol und deren Salze und Ester und Mischungen davon besteht, ausgewählt.

Die Osmolalitätsregler werden vorzugsweise aus der Gruppe, die aus Sorbitol, Glukose, Glycerin, Polyethylenglycol, Fruktose und Mischungen davon besteht, ausgewählt.

Als Lösungsmittel können beispielsweise Wasser, einwertige Alkohole, Paraffine, Triglyceride, Öle oder Mischungen davon verwendet werden.

Gemäß einer Weiterbildung kann die erfindungsgemäße phosphatfreie Zusammensetzung auch Solubilisatoren, Detergenzien und/oder Emulgatoren enthalten, um die Löslichkeit des wenigstens einen hydrophoben FP-Prostanoidrezeptor-Agonistens und/oder Prostamidrezeptor-Agonistens weiter zu verbessern. Um eine unerwünschte Reizung des Auges zu vermeiden, werden gemäß einer bevorzugten Variante möglichst geringfügige Mengen an Solubilisatoren, Detergenzien und/oder Emulgatoren hinzugefügt.

Vorzugsweise wird die erfindungsgemäße pharmazeutische Zusammensetzung konservierungsmittelfrei formuliert. Um eine unerwünschte Reizung des Auges zu vermeiden, werden gemäß einer bevorzugten Variante keine Konservierungsstoffe, insbesondere kein Benzalkoniumchlorid, zu der erfindungsgemäßen pharmazeutischen Zusammensetzung hinzugefügt.

Weiterhin ist erfindungsgemäß bevorzugt, daß die phosphatfreie pharmazeutische Zusammensetzung in Form einer Lösung, von Tropfen, eines Sprays, einer Suspension, Emulsion, eines Gels, einer Salbe, Paste, eines Puders, Pulvers, Granulats oder einer Tablette vorliegt.

Die phosphatfreie pharmazeutische Zusammensetzung ist bevorzugt ein Ophthalmikum, weiter bevorzugt ein Ophthalmikum zur topischen Anwendung.

Bei der Bereitstellung der phosphatfreien pharmazeutischen Zusammensetzung in Form von Augensalben bzw. Augengelen wird diese bspw. in Vaseline oder Paraffin mit und ohne Emulgatorzusatz wie bspw. Cholesterin, Wollwachs, Wollwachsalkohole, Cetanol etc. bereitgestellt.

Gemäß einer bevorzugten Ausführungsform liegt die phosphatfreie pharmazeutische Zusammensetzung in der Form einer, vorzugsweise wässrigen, Lösung vor, so daß diese bspw. in der Form von Augentropfen oder eines Augensprays auf die Oberfläche des Auges aufgebracht werden kann.

Bei einer Ausführungsform liegt die Osmolarität der erfindungsgemäßen phosphatfreien pharmazeutischen Zusammensetzung bei 100 bis 900 mOsm/l.

Die vorzugsweise wäßrigen Lösungen sind dabei gemäß einer bevorzugten Ausführungsform, bezogen auf die Tränenflüssigkeit, isotone Lösungen. Bei isotonen Lösungen liegt die Osmolarität vorzugsweise bei 200 bis 350 mOsm/l, vorzugsweise bei 300 mOsm/l. Gemäß einer weiteren bevorzugten Ausführungsform ist die erfindungsgemäße phosphatfreie pharmazeutische Zusammensetzung hypoosmolar. In diesem Fall kann die Osmolarität beispielsweise etwa 160-180 mOsm/l betragen. Eine hypoosmolare Lösung findet insbesondere dann Anwendung, wenn eine abnorm hohe Osmolarität eines Tränenfilms bei einem Patienten mit trockenen Augen ausgeglichen werden muß. In Abhängigkeit von dem zu behandelnden Krankheitsbild kann auch eine hypertone Lösung vorteilhaft sein. Die pharmazeutische Zusammensetzung kann dabei auch eine besonders hohe Osmolarität von 700 bis 900 mOsm/l.

Zur Isotonisierung der wäßrigen Lösung werden bevorzugt Natriumchlorid, Borsäure, Sorbitol, Glycerin, etc. verwendet.

Die erfindungsgemäße phosphatfreie Zusammensetzung eignet sich auch zur Herstellung eines Kombinationspräparats, wobei das/die FP-Prostanoid(e) und/oder Prostamid(e) mit einem oder mehreren weiteren Wirkstoffen gemeinsam vorliegen. Somit betrifft die vorliegende Erfindung auch eine pharmazeutische Zusammensetzung in Form eines Kombinationspräparates, bei dem neben FP-Prostanoid und/oder Prostamid wenigstens ein weiterer Wirkstoff oder wenigstens zwei weitere Wirkstoffe vorliegen.

Gemäß einer bevorzugten Weiterbildung der Erfindung kann die erfindungsgemäße pharmazeutische Zusammensetzung neben FP-Prostanoid (en) (FP-Prostanoidrezeptor-Agonist(en)) und/oder Prostamid(en) (Prostamidrezeptor-Agon ist(en)) auch Betablocker, Carboanhydrasehemmer, Sympathomimetika, Parasympathomimetika oder Mischungen davon enthalten.

Bei den vorgenannten FP-Prostanoiden handelt es sich insbesondere um Latanoprost, Travoprost und/oder Tafluprost, und bei den vorgenannten Prostamiden handelt es sich insbesondere um Bimatoprost, die jeweils mit den nachstehend genannten Wirkstoffen als erfindungsgemäße Wirkstoffkombination vorliegen können.

Als Betablocker haben sich insbesondere Timolol, Betaxolol, Carteolol, Levobunolol als auch deren Salze und/oder Ester oder Mischungen davon als sehr geeignet erwiesen.

Das Timolol wird vorzugsweise in Form von Timololhydrogenmaleat verwendet.

Als Carboanhydrasehemmer haben sich insbesondere Dorzolamid, Brinzolamid, Acetazolamid als auch deren Salze und/oder Ester oder Mischungen davon als sehr geeignet erwiesen.

Das Dorzolamid wird vorzugsweise in Form des Hydrochlorids (Dorzolamid-HCl) verwendet.

Als Sympathomimetika haben sich insbesondere Brimonidin, Apraclonidin (Iopidine), Acetazolamid als auch deren Salze und/oder Ester oder Mischungen davon als sehr geeignet erwiesen.

Das Brimonidin wird vorzugsweise als Tartratsalz (Brimonidin[(R,R)-tartrat], z.B. Alphagan, verwendet.

Als Parasympathomimetikum hat sich Pilocarpin als auch dessen Salze und/oder Ester oder Mischungen davon als sehr geeignet erwiesen.

Das Pilocarpin wird vorzugsweise in Form von Pilocarpinnitrat verwendet.

Gemäß einer bevorzugten Variante der Erfindung umfasst die Wirkstoffkombination FP-Prostanoid und Timolol oder Prostamid und Timolol:
- Latanoprost und Timolol
- Travoprost und Timolol
- Tafluprost und Timolol
- Bimatoprost und Timolol

Gemäß einer bevorzugten Variante der Erfindung umfasst die Wirkstoffkombination FP-Prostanoid und Timolol oder Prostamid und Dorzolamid:
- Latanoprost und Dorzolamid
- Travoprost und Dorzolamid
- Tafluprost und Dorzolamid
- Bimatoprost und Dorzolamid

Die vorstehenden Wirkstoffkombinationen können selbstverständlich Hilfsstoffe wie beispielsweise anorganische Puffersubstanzen, organische Puffersubstanzen, anorganische Salze, organische Salze, Emulgatoren, Solubilisatoren, Benetzungsmittel, Spreitmittel, Antioxidationsmittel, Osmolalitätsregler, etc. oder Mischungen davon enthalten.
Die erfindungsgemäße phosphatfreie pharmazeutische Zusammensetzung kann bei der Therapie und/oder Prävention von Glaukom verwendet werden.

Insbesondere eignet sich die erfindungsgemäße phosphatfreie pharmazeutische Zusammensetzung zur Herstellung eines Medikamentes zur Therapie und/oder Prävention von Glaukom.

Die erfindungsgemäße pharmazeutische Zusammensetzung liegt vorzugsweise in einem Aufbewahrungs- und Dosierungssystem vor. Vorzugsweise handelt es sich dabei um ein Mehrdosenbehälter oder Mehrdosensystem. Als sehr geeignet hat sich hierfür das COMOD^{®}-System der Fa. Ursapharm, Saarbrücken, Deutschland erwiesen. Gemäß einer bevorzugten Variante besteht der Behälter, in dem die erfindungsgemäße pharmazeutische Zusammensetzung gelagert ist, zu wenigstens aus 75 Gew.-% Polyethylen, vorzugsweise etwa 78 bis 94 Gew.-%, Polyethylen, jeweils bezogen auf das Gesamtgewicht des Behälters. Der Rest zu 100 Gew.-% ist vorzugsweise ein von Polyethylen verschiedenes Alken oder Polyalken.

Gemäß einer weiter bevorzugten Variante besteht der Behälter, in dem die erfindungsgemäße pharmazeutische Zusammensetzung gelagert ist, zu wenigstens aus 75 Gew.-% Polypropylen, vorzugsweise etwa 78 bis 94 Gew.-%, Polypropylen, jeweils bezogen auf das Gesamtgewicht des Behälters. Der Rest zu 100 Gew.-% ist vorzugsweise ein von Polypropylen verschiedenes Alken oder Polyalken.

Gemäß einer weiter bevorzugten Variante besteht der Behälter aus Glas.

Die Erfindung wird nachfolgend anhand von Beispielen erläutert, ohne darauf beschränkt zu sein.

### Beispiele

**Beispiel 1)**

| | |
|---|---|
| Bimatoprost | 0, 3mg |
| Polyvinylpyrrolidon(Kollidon) | 20,0 mg |
| Citronensäure | 0,05 mg |
| Natriumcitrat x 2 H₂O | 8, 5 mg |
| Sorbit | 33 mg |
| Wasser für Injektionszwecke (f. 1.) | ad 1,0 ml |

eingestellt auf pH 7,4 mit 0,1 N NaOH

**Beispiel 2)**

| | |
|---|---|
| Bimatoprost | 0,3 mg |
| Hyaluronsäure, Na-Salz (MG: 2-3 • 10⁶ Da) | 0,1 mg |
| Citronensäure | 0,05 mg |
| Natriumcitrat x 2 H₂O | 8,5 mg |
| Sorbit | 33 mg |
| Wasser f. I. | ad 1,0 ml |

eingestellt auf pH 7,4 mit 0,1 N NaOH

**Beispiel 3)**

| | |
|---|---|
| Travoprost | 0,04 mg |
| Hyaluronsäure, Na-Salz (MG: 2-3 • 10⁶ Da) | 0,1 mg |
| Sorbit | 30 mg |
| Natriumcitrat x 2 H₂O | 15 mg |
| Citronensäure | 0,65 mg |
| Wasser f. I. | ad 1,0 ml |

eingestellt auf pH 6,0 mit 0,1 N HCl

**Beispiel 4)**

| | |
|---|---|
| Latanoprost | 0,05 mg |
| Hyaluronsäure, Na-Salz(MG: 2-3 • 10⁶ Da) | 0,1 mg |
| Sorbit | 30 mg |
| Natriumcitrat x 2 H₂O | 15 mg |
| Citronensäure | 0,65 mg |
| Wasser f. I. | ad 1,0 ml |

eingestellt auf pH 6,0 mit 0,1 N HCl

**Beispiel 5)**

| | |
|---|---|
| Latanoprost | 0,05 mg |
| Polyvinylpyrrolidon (Kollidon) | 20,0 mg |
| Natriumcitrat x 2 H₂O | 7,5 mg |
| Citronensäure | 0,2 mg |
| Natriumchlorid | 6,0 mg |
| Wasser f. I. | ad 1,0 ml |

eingestellt auf pH 6,0 mit 0,1 N HCl

**Beispiel 6)**

| | |
|---|---|
| Tafluprost | 0,015 mg |
| Polyvinylpyrrolidon (Kollidon) | 20,0 mg |
| Natriumcitrat x 2 H₂O | 7,5 mg |
| Citronensäure | 0,2 mg |
| Natriumchlorid | 6,0 mg |
| Wasser f. I. | ad 1,0 ml |

eingestellt auf pH 6,0 mit 0,1 N HCl

**Beispiel 7)**

| | |
|---|---|
| Tafluprost | 0,05 mg |
| Hyaluronsäure, Na-Salz (MG: 2-3 • 10⁶ Da) | 1,0 mg |
| Natriumcitrat x 2 H₂O | 7,5 mg |
| Citronensäure | 0,2 mg |
| Natriumchlorid | 6,0 mg |
| Wasser, f. I. | ad 1,0 ml |

eingestellt auf pH 6 mit 0,1 N HCl

**Beispiel 8)**

| | |
|---|---|
| Latanoprost | 0,05 mg |
| Natriumchlorid | 6,0 mg |
| Natriumcitrat x 2 H₂O | 10,0 mg |
| Citronensäure | 0,2 mg |
| Wasser f. I. | ad 1,0 ml |

eingestellt auf pH 6,0 mit 0,1 N HCl

**Beispiel 9)**

| | |
|---|---|
| Latanoprost | 0,05 mg |
| Natriumchlorid | 6,0 mg |
| Natriumcitrat x 2 H₂O | 10,0 mg |
| Citronensäure | 0,2 mg |
| Wasser f. I. | ad 1,0 ml |

eingestellt auf pH 6,0 mit 0,1 N HCl

**Beispiel 10)**

| | |
|---|---|
| Latanoprost | 0,05 mg |
| Natriumchlorid | 6,0 mg |
| Natriumcitrat x 2 H₂O | 10,0 mg |
| Citronensäure | 0,2 mg |
| Wasser f. I. | ad 1,0 ml |

eingestellt auf pH 6,0 mit 0,1 N HCl

**Beispiel 11)**

| | |
|---|---|
| Bimatoprost | 0,3 mg |
| Timolol | 2,5 mg |
| Hyaluronsäure, Na-Salz (MG: 2-3 • 10⁶ Da) | 0,1 mg |
| Citronensäure | 0,05 mg |
| Natriumcitrat x 2 H₂O | 8,5 mg |
| Sorbit | 33 mg |
| Wasser f. I. | ad 1,0 ml |

eingestellt auf pH 7,4 mit 0,1 N NaOH

**Beispiel 12)**

| | |
|---|---|
| Bimatoprost | 0,3mg |
| Dorzolamid | 10 mg |
| Hyaluronsäure, Na-Salz (MG: 2-3 • 10⁶ Da) | 0,1 mg |
| Citronensäure | 0,05 mg |
| Natriumcitrat x 2 H₂O | 8,5 mg |
| Sorbit | 33 mg |
| Wasser f. I. | ad 1,0 ml |

eingestellt auf pH 7,4 mit 0,1 N NaOH

**Beispiel 13)**

| | |
|---|---|
| Travoprost | 0,04 mg |
| Timolol | 2,5 mg |
| Hyaluronsäure, Na-Salz (MG: 2-3 • 10⁶ Da) | 0,1 mg |
| Sorbit | 30 mg |
| Natriumcitrat x 2 H₂O | 15 mg |
| Citronensäure | 0,65 mg |
| Wasser f. I. | ad 1,0 ml |

eingestellt auf pH 6,0 mit 0,1 N HCl

**Beispiel 14)**

| | |
|---|---|
| Travoprost | 0,04 mg |
| Dorzolamid | 10 mg |
| Hyaluronsäure, Na-Salz (MG: 2-3 • 10⁶ Da) | 0,1 mg |
| Sorbit | 30 mg |
| Natriumcitrat x 2 H₂O | 15 mg |
| Citronensäure | 0, 65 mg |
| Wasser f. I. | ad 1,0 ml |

eingestellt auf pH 6,0 mit 0,1 N HCl

**Beispiel 15)**

| | |
|---|---|
| Latanoprost | 0,05 mg |
| Timolol | 2,5 mg |
| Hyaluronsäure, Na-Salz (MG: 2-3 • 10⁶ Da) | 0,1 mg |
| Sorbit | 30 mg |
| Natriumcitrat x 2 H₂O | 15 mg |
| Citronensäure | 0,65 mg |
| Wasser f. I. | ad 1,0 ml |

eingestellt auf pH 6,0 mit 0,1 N HCl

**Beispiel 16)**

| | |
|---|---|
| Latanoprost | 0,05 mg |
| Dorzolamid | 10 mg |
| Hyaluronsäure, Na-Salz (MG: 2-3 • 10⁶ Da) | 0,1 mg |
| Sorbit | 30 mg |
| Natriumcitrat x 2 H₂O | 15 mg |
| Citronensäure | 0,65 mg |
| Wasser f. I. | ad 1,0 ml |

eingestellt auf pH 6,0 mit 0,1 N HCl

**Beispiel 17)**

| | |
|---|---|
| Tafluprost | 0,05 mg |
| Timolol | 2,5 mg |
| Hyaluronsäure, Na-Salz (MG: 2-3 • 10⁶ Da) | 1,0 mg |
| Natriumcitrat x 2 H₂O | 7,5 mg |
| Citronensäure | 0,2 mg |
| Natriumchlorid | 6,0 mg |
| Wasser, f. I. | ad 1,0 ml |

eingestellt auf pH 6 mit 0,1 N HCl

**Beispiel 18)**

| | |
|---|---|
| Tafluprost | 0,05 mg |
| Dorzolamid | 10 mg |
| Hyaluronsäure, Na-Salz (MG: 2-3 • 10⁶ Da) | 1,0 mg |
| Natriumcitrat x 2 H₂O | 7,5 mg |
| Citronensäure | 0,2 mg |
| Natriumchlorid | 6,0 mg |
| Wasser, f. I. | ad 1,0 ml |

eingestellt auf pH 6 mit 0,1 N HCl

## Patentansprüche

1. Phosphatfreie pharmazeutische Zusammensetzung, enthaltend wenigstens einen
a) FP-Prostanoidrezeptor-Agonisten ausgewählt aus der Gruppe bestehend aus Prostaglandin F₂ₐₗₚₕₐ, sowie den Prostaglandin F₂ₐₗₚₕₐ-Analoga Latanoprost, Tafluprost, Travoprost, Unoproston, dem 15-Keto-Analogon vcn Latanoprost, dem 15-Keto-Analogon von Travoprost und Mischungen davon sowie deren annehmbaren Salzen und Estern, und/oder
b) Prostamidrezeptor-Agonisten, ausgewählt aus der Gruppe bestehend aus Prostamid, Prostaglandin F₂ₐₗₚₕₐ-amid, 15-Keto-Prostaglandin F₂ₐₗₚₕₐ-amid-Analogon oder ein pharmazeutisch annehmbares Salz hiervon oder ein pharmazeutisch annehmbarer Ester hiervon, sowie Citratsalze und/oder Citronensäure zur Anwendung in einen Verfahren zur
a) Verhinderung oder Prophylaxe der Ab- oder Einlagerung von schwer löslichen Calcium-Verbindungen auf oder in der Hornhaut und/oder der Bindehaut des Auges,
b) Prophylaxe der Calcifikation und/oder Verkalkung der Hornhaut und/oder der Bindehaut des Auges,
bei der Therapie von Glaukon.

2. Phosphatfreie pharmazeutische Zusammensetzung zur Anwendung nach Anspruch 1,
**dadurch gekennzeichnet,**
**dass** die Bildung schwer löslicher Calcium-Verbindungen, Calcium-Phosphat-Komplexe und/oder Calcium-Phosphat-Verbindungen verhindert wird.

3. Phosphatfreie pharmazeutische Zusammensetzung zur Anwendung nach einem der vorhergehenden Ansprüche,
**dadurch gekennzeichnet,**
**dass** das Prostaglandin F₂ₐₗₚₕₐ-amid die chemische Strukturformel (VIII) 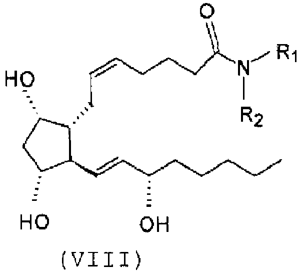 aufweist,
wobei R₁ und R₂ unabhängig voneinander für Wasserstoff, Alkyl oder Hydroxyalkyl mit 1 bis 8 Kohlenstoffatomen, vorzugsweise mit 2 bis 4 Kohlenstoffatomen, stehen.

4. Phosphatfreie pharmazeutische Zusammensetzung zur Anwendung nach Anspruch 3,
**dadurch gekennzeichnet,**
**dass** R₁ für Alkyl oder Hydroxyalkyl mit 1 bis 8 Kohlenstoffatomen, vorzugsweise mit 2 bis 4 Kohlenstoffatomen, steht und R₂ für Wasserstoff steht.

5. Phosphatfreie pharmazeutische Zusammensetzung zur Anwendung nach einem der vorhergehenden Ansprüche,
**dadurch gekennzeichnet,**
**dass** das Prostaglandin F₂ₐₗₚₕₐ-amid Prostaglandin F₂ₐₗₚₕₐ-1-ethanolamid oder Bimatoprost ist.

6. Phosphatfreie pharmazeutische zusammensetzung zur Anwendung nach einem der vorherigen Ansprüche,
**dadurch gekennzeichnet,**
**dass** die pharmazeutische Zusammensetzung mindestens einen ophthalmologisch verträgliche Viskositätsregler umfasst, der bevorzugt ausgewählt ist aus der Gruppe bestehend aus Chondroitinsulfat, Polyacrylamid, Polyacrylsäure, Polyacrylharze, Polyethylenglykol, Cellulosederivate, Polysaccharide, Polyvinylpyrrolidon, Hyaluronsäure, Hyaluronate, Derivaten davon, wobei die Hyaluronsäure, das Hyaluronat und/oder deren Derivate insbesondere ein Molekulargewicht aufweisen, das in einem Bereich von etwa 50.000 bis etwa 10.000.000 Dalton, bevorzugt von etwa 250.000 bis etwa 5.000.000 Dalton, liegt.

7. Phosphatfreie pharmazeutische Zusammensetzung zur Anwendung nach einem der vorhergehenden Ansprüche,
**dadurch gekennzeichnet,**
**dass** die pharmazeutische Zusammensetzung phosphatfreie pharmazeutische Hilfsstoffe, die aus der Gruppe, die aus anorganischen Puffersubstanzen, organischen Puffersubstanzen, anorganischen Salzen, organischen Salzen, Lösungsmitteln, Lösungsvermittlern, Lösungsbeschleunigern, Salzbildnern, Viskositäts- und Konsistenzbeeinflussern, Gelbildnern, Emulgatoren, Solubilisatoren, Benetzern, Spreizmitteln, Anti-Oxidantien, Konservierungsmitteln, Füll- und Trägerstoffen, Osmolaritätsreglern sowie Mischungen davon besteht, ausgewählt werden, enthält, wobei die pharmazeutische Zusammensetzung auch konservierungsmittelfrei ausgebildet sein kann.

8. Phosphatfreie pharmazeutische Zusammensetzung zur Anwendung nach einem der vorhergehenden Ansprüche,
**dadurch gekennzeichnet,**
**dass** die phosphatfreie pharmazeutische Zusammensetzung in Form einer Lösung, von Tropfen, eines Sprays, einer Suspension, Emulsion, eines Gels, einer Salbe, Paste, eines Puders, Pulvers, Granulats oder einer Tablette vorliegt.

9. Phosphatfreie pharmazeutische Zusammensetzung zur Anwendung nach einem der vorhergehenden Ansprüche, **dadurch gekennzeichnet, dass** sie in einem Aufbewahrungs- und/oder Dosierungssystem, insbesondere in Form eines Mehrdosenbehälters oder Mehrdosensystems, enthalten ist.

## Claims

1. A phosphate-free pharmaceutical composition, containing at least one
a) FP-prostanoid receptor agonist selected from the group consisting of prostaglandin F₂ₐₗₚₕₐ, and the prostaglandin F₂ₐₗₚₕₐ analogues latanoprost, tafluprost, travoprost, unoprostone, the 15-keto analogue of latanoprost, the 15-keto analogue of travoprost and mixtures thereof and also their acceptable salts and esters, and/or
b) prostamide receptor agonists, selected from the group consisting of prostamide, prostaglandin F₂ₐₗₚₕₐ amide, 15-keto-prostaglandin F₂ₐₗₚₕₐ amide analogue or a pharmaceutically acceptable salt thereof or a pharmaceutically acceptable ester thereof,
and also citrate salts and/or citric acid for use in a method for
a) prevention or prophylaxis of the deposition or inclusion of poorly soluble calcium compounds on or in the cornea and/or the conjunctiva of the eye,
b) prophylaxis of the calcification and/or calcinosis of the cornea and/or the conjunctiva of the eye,
in the treatment of glaucoma.

2. A phosphate-free pharmaceutical composition for use according to Claim 1,
**characterised in that**
the formation of poorly soluble calcium compounds, calcium phosphate complexes and/or calcium phosphate compounds is prevented.

3. A phosphate-free pharmaceutical composition for use according to one of the preceding claims,
**characterised in that**
the prostaglandin F₂ₐₗₚₕₐ amide has the chemical structural formula (VIII) 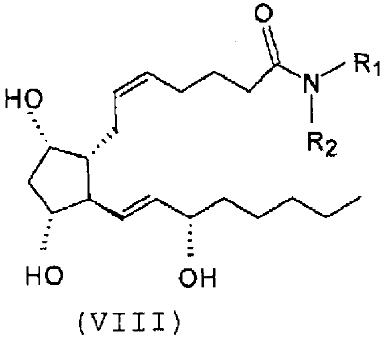 wherein R₁ and R₂ independently of each other stand for hydrogen, alkyl or hydroxyalkyl with 1 to 8 carbon atoms, preferably with 2 to 4 carbon atoms.

4. A phosphate-free pharmaceutical composition for use according to Claim 3,
**characterised in that**
that R₁ stands for alkyl or hydroxyalkyl with 1 to 8 carbon atoms, preferably with 2 to 4 carbon atoms, and R₂ stands for hydrogen.

5. A phosphate-free pharmaceutical composition for use according to one of the preceding claims,
**characterised in that**
the prostaglandin F₂ₐₗₚₕₐ amide is prostaglandin F₂ₐₗₚₕₐ-1-ethanolamide or bimatoprost.

6. A phosphate-free pharmaceutical composition for use according to one of the preceding claims,
**characterised in that**
the pharmaceutical composition comprises at least one ophthalmologically compatible viscosity stabiliser which is preferably selected from the group consisting of chondroitin sulfate, polyacrylamide, polyacrylic acid, polyacrylic resins, polyethylene glycol, cellulose derivatives, polysaccharides, polyvinylpyrrolidone, hyaluronic acid, hyaluronates, derivatives thereof, the hyaluronic acid, the hyaluronate and/or derivatives thereof having in particular a molecular weight which lies in a range from about 50,000 to approximately 10,000,000 dalton, preferably from approximately 250,000 to approximately 5,000,000 dalton.

7. A phosphate-free pharmaceutical composition for use according to one of the preceding claims,
**characterised in that**
the pharmaceutical composition contains phosphate-free pharmaceutical auxiliaries which are selected from the group consisting of inorganic buffer substances, organic buffer substances, inorganic salts, organic salts, solvents, solubility aids, solubility promoters, salt-formers, viscosity and consistency regulators, gelling agents, emulsifiers, solubilisers, wetting agents, expanding agents, antioxidants, preservatives, fillers and excipients, osmolarity regulators and mixtures thereof, wherein the pharmaceutical composition may also be formed without preservatives.

8. A phosphate-free pharmaceutical composition for use according to one of the preceding claims,
**characterised in that**
the phosphate-free pharmaceutical composition is in the form of a solution, drops, a spray, a suspension, emulsion, a gel, an ointment, paste, a fine powder, coarse powder, granules or a tablet.

9. A phosphate-free pharmaceutical composition for use according to one of the preceding claims, **characterised in that** it is contained in a storage and/or metering system, in particular in the form of a multi-dose container or multi-dose system.

## Revendications

1. Composition pharmaceutique sans phosphate, contenant au moins
a) un agoniste des récepteurs des prostanoïdes FP, choisi dans le groupe consistant en la prostaglandine F₂ₐₗₚₕₐ, ainsi que les analogues de la prostaglandine F₂ₐₗₚₕₐ latanoprost, tafluprost, travoprost, unoprostone, l'analogue 15-céto du latanoprost, l'analogue 15-céto du travoprost, et les mélanges de ceux-ci, ainsi que les sels et esters acceptables de ceux-ci, et/ou
b) les agonistes des récepteurs des prostamides, choisis dans le groupe consistant en le prostamide, l'amide de la prostaglandine F₂ₐₗₚₕₐ, les analogues de l'amide de la 15-céto-prostaglandine F₂ₐₗₚₕₐ, ou un sel pharmaceutiquement acceptable de ceux-ci, ou un ester pharmaceutiquement acceptable de ceux-ci, ainsi que les sels citrates et/ou l'acide citrique,
pour utilisation dans un procédé destiné
a) à la prévention ou à la prophylaxie du dépôt ou de l'inclusion de composés du calcium difficilement solubles sur ou dans la cornée et/ou la conjonctive de l'oeil,
b) à la prophylaxie de la calcification et/ou de la dégénérescence calcaire de la cornée et/ou de la conjonctive de l'oeil,
lors du traitement du glaucome.

2. Composition pharmaceutique sans phosphate pour l'utilisation selon la revendication 1, **caractérisée en ce qu'**est prévenue la formation de composés du calcium difficilement solubles, de complexes de phosphate de calcium et/ou de composés du phosphate de calcium.

3. Composition pharmaceutique sans phosphate pour l'utilisation selon l'une des revendications précédentes, **caractérisée en ce que** le prostaglandine F₂ₐₗₚₕₐ-amide présente la formule développée chimique (VIII) 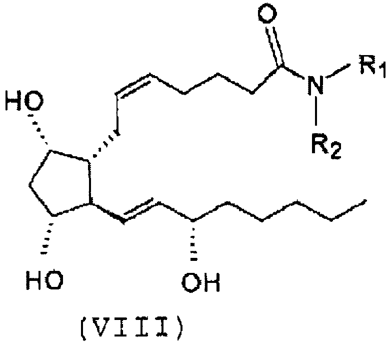 dans laquelle R₁ et R₂ représentent chacun indépendamment de l'autre un atome d'hydrogène, un groupe alkyle ou hydroxyalkyle ayant 1 à 8 atomes de carbone, de préférence 2 à 4 atomes de carbone.

4. Composition pharmaceutique sans phosphate pour l'utilisation selon la revendication 3, **caractérisée en ce que** R₁ représente un groupe alkyle ou hydroxyalkyle ayant 1 à 8 atomes de carbone, de préférence 2 à 4 atomes de carbone, et R₂ représente un atome d'hydrogène.

5. Composition pharmaceutique sans phosphate pour l'utilisation selon l'une des revendications précédentes, **caractérisée en ce que** l'amide de la prostaglandine F₂ₐₗₚₕₐ est le 1-éthanolamide de la prostaglandine F₂ₐₗₚₕₐ ou le bimatoprost.

6. Composition pharmaceutique sans phosphate pour l'utilisation selon l'une des revendications précédentes, **caractérisée en ce que** la composition pharmaceutique comprend au moins un régulateur de viscosité, compatible d'un point de vue ophtalmologique, qui de préférence est choisi dans le groupe consistant en le sulfate de chondroïtine, le polyacrylamide, le poly(acide acrylique), les résines polyacryliques, le polyéthylèneglycol, les dérivés de la cellulose, les polysaccharides, la polyvinylpyrrolidone, l'acide hyaluronique, les hyaluronates, les dérivés de ceux-ci, auquel cas l'acide hyaluronique, l'hyaluronate et/ou les dérivés de ceux-ci présentent en particulier une masse moléculaire qui est comprise dans une plage d'environ 50 000 à environ 10 000 000 Daltons, de préférence d'environ 250 000 à environ 5 000 000 Daltons.

7. Composition pharmaceutique sans phosphate pour l'utilisation selon l'une des revendications précédentes, **caractérisée en ce que** la composition pharmaceutique contient des adjuvants pharmaceutiques sans phosphate, qui sont choisis dans le groupe consistant les substances tampon inorganiques, les substances tampon organiques, les sels inorganiques, les sels organiques, les solvants, les tiers-solvants, les accélérateurs de dissolution, les agents de salification, les agents influant sur la viscosité et la consistance, les gélifiants, les émulsifiants, les solubilisateurs, les mouillants, les agents d'étalement, les antioxydants, les conservateurs, les matières de charge et supports, les régulateurs d'osmolarité, ainsi que les mélanges de ceux-ci, la composition pharmaceutique pouvant aussi être configurée sans conservateur.

8. Composition pharmaceutique sans phosphate pour l'utilisation selon l'une des revendications précédentes, **caractérisée en ce que** la composition pharmaceutique sans phosphate se présente sous forme d'un soluté, de gouttes, d'un spray, d'une suspension, d'une émulsion, d'un gel, d'une pommade, d'une pâte, d'une poudre, d'une poudre fine, d'un granule ou d'un comprimé.

9. Composition pharmaceutique sans phosphate pour l'utilisation selon l'une des revendications précédentes, **caractérisée en ce qu'**elle est contenue dans un système de conservation et/ou de dosage, en particulier sous forme d'un récipient multidose ou d'un système multidose.
